Europäisches Patentamt

European Patent Office

Office européen des brevets

(19)

(11) Publication number: **0 253 270 B1**

# EUROPEAN PATENT SPECIFICATION

(12)

(45) Date of publication of patent specification: **23.09.92**  (51) Int. Cl.5: **G01N 33/538**, G01N 33/543

(21) Application number: **87109740.8**

(22) Date of filing: **07.07.87**

(54) **Method for diagnostic immunoassay by solid phase separation.**

(30) Priority: **14.07.86 US 885130**

(43) Date of publication of application:
**20.01.88 Bulletin  88/03**

(45) Publication of the grant of the patent:
**23.09.92 Bulletin  92/39**

(84) Designated Contracting States:
**AT BE CH DE ES FR GB IT LI NL**

(56) References cited:
EP-A- 0 095 932      EP-A- 0 143 274
EP-A- 0 158 443      EP-A- 0 285 995
US-A- 3 654 090      US-A- 3 853 987
US-A- 4 298 687

MARCEL DEKKER INCORP., 1984, Marcel Dekker, New York, NY (US); M.ZOUHAIR ATASSI et al, pp. 361-362

(73) Proprietor: **ABBOTT LABORATORIES**
**14th Street and Sheridan Road North St**
**North Chicago, Illinois 60064(US)**

(72) Inventor: **Pry, Terry Alan**
**219 Butterfield Road**
**Libertyville, IL 60048(US)**
Inventor: **Granados, Edward Nicholas**
**19 Montgomery Lane**
**Vernon Hills, IL 60061(US)**

(74) Representative: **Modiano, Guido et al**
**MODIANO, JOSIF, PISANTY & STAUB**
**Modiano & Associati Via Meravigli, 16**
**I-20123 Milano(IT)**

## Description

Background of the Invention

The present invention is directed towards a method for performing a diagnostic immunoassay for the measurement of small and large proteins and analytes in biological fluids by a solid-phase separation. This method is especially suitable for use in automated systems.

Many diagnostic immunoassays are known which generally employ the specific binding characteristics that exist between an analyte or protein with a specific antibody tagged with some traceable substituent. One problem which has long been associated with this method is how to remove excess antibody from the biological fluid being tested for concentration of analyte in a manner whereby the analyte concentrations can be accurately measured.

Various attempts to remove excess antibody include U.S. Patent 4,298,687 which discloses the absorption of unreacted antibody on a solid phase consisting of a polyacrylamide gel sensitized to the specific antibody.

U.S. Patent 4,551,426 discloses another method to remove excess antibody in an immunoassay for digoxin. Here, excess labeled antobody is removed by passing it through an affinity column that has ouabain, an analog of digoxin, immobilized on the solid phase chromatography matrix. The excess antibody is absorbed by the oubain. The chromatography elute is then examined for the labeled antibody-analyte complex.

While the above can be effective, they are all subject to improvement, especially with respect to ease in operation, handling and thereby accuracy. The subject method provides improvements over these methods and is especially adaptable to automated analysis which is not the case with many of the known methods.

Summary of the Invention

The present invention is directed towards a method for conducting a diagnostic immunoassay comprising the steps of:

a) forming, in liquid phase, a reaction mixture of a test sample with a molar excess of labeled antibody whereby said labeled antibody is capable of forming a complex with analyte present in said test sample;

b) contacting said reaction mixture with a solid phase material having immobilized thereon a compound in an amount capable of complexing with any of said excess labeled antibody, to form a solid phase complex;

c) separating said liquid phase from said solid phase material and any complexes thereof; and

d) measuring the amount of complex present in said liquid phase,

characterized in that said solid phase material has a rate of settling by gravity in the range of 5 seconds to 2 minutes per centimeter in water and said separation of step (c) is accomplished by settling of the solid phase material by gravity.

Preferably the solid phase material is from about 5 to about 300 $\mu$m in diameter. The solid phase material can be formed of any of a variety of materials, preferably, agarose, polystyrene, polyacrylamide, their derivatives or mixtures thereof.

The solid phase material has immobilized thereon a compound capable of binding the excess labeled antibody such as a corresponding antigen or chemical analogue. Typically, the labeled antibody is an enzyme labeled antibody.

The present method is particularly adapted for use in automated diagnostic apparatus where centrifugation filtration or column filtration is not possible or practical.

Brief Description of the Drawing

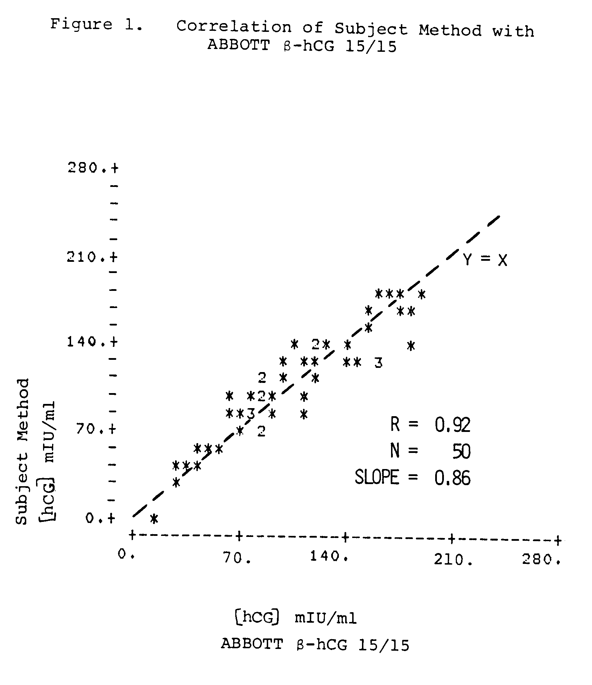

Figure 1 is a graph of the correlation of the data obtained with the subject method versus a standard method (Abbot $\beta$-hCG 15/15).

Detailed Description of the Invention

The present invention provides for a method whereby any excess labeled compound employed to identify a particular analyte can be readily removed to permit measurement of the analyte. The method, as outlined below, is particularly adapted for use in automated systems where traditional purification or removal means are not appropriate, i.e., filtration, centrifugation or filtration columns. More preferably the subject method is employed with the diagnostic immunoassay apparatus TDx™, a trademark of Abbott Laboratories for an automated system for the quantitation of therapeutic drug concentrations in serum or plasma based on the method of fluorescence polarization immunoassay.

Generally, the method comprises the preparation of a reaction mixture of a biological fluid suspected of containing a known analyte, or protein with a labeled substance, antibody, capable of complexing with the particular analyte, protein or material sought to be quantified. The labeled substance is added to the reaction mixture in a molar excess with regard to the suspected amount of material to be quantified to assure complete complexing thereof.

After an appropriate incubation time, i.e., sufficient time to allow the labeling substance to complex with all the material sought to be quantified, the complex is measured. However, because the labeling substance is employed in a molar excess it is necessary to remove this excess prior to measuring the amount of complex formed.

The subject method is characterized by removing the excess labeled substance by adding a solid phase material having immobilized thereon a compound capable of complexing with the labeled substance. The solid phase material is of such physical characteristics that it can be easily dispersed in the reaction mixture and settled out. Therefore the density and overall size of the solid phase material is such that a rapid disperse by agitation and sediment by gravity is facilitated.

The solid phase material is present as a particle or matrix having a density greater than water. The solid phase material has a sedimentation rate in water of from about 5 seconds per centimeter to about 2 minutes per centimeter, more preferably from about 20 seconds per centimeter to about 1.5 minutes per centimeter. The size of the particle which forms the solid phase material can be from about 5 $\mu$m to about 300 $\mu$m, preferably from about 40 $\mu$m to about 120 $\mu$m in diameter. The sedimentation rate and size is important to assuring the solid phase material both disperses and settles readily without significant input of energy or motion to the reaction vessel containing the subject reaction mixture. This is especially important when employing automated diagnostic apparatus.

The solid phase material can be fabricated from any number of material or synthetic materials. Preferably the solid phase material is manufactured from polymeric materials such as agarose, polystyrene, polyacrylamide, their derivatives or mixtures thereof. Generally the solid phase is present as bead-like structures. The solid phase material can be delivered to the reaction vessel as a dry powder, wet slurry, tablet or a capsule.

Immobilized on the solid phase material is a compound capable of complexing the labeled substance. Generally this is a complementary substance to the substance to be identified. Examples include an analyte, or its analog. The complex formed is irreversibly bonded to the solid phase material and because of its physical characteristics settles out or sediments by gravity. The total reaction mixture thus becomes separated into a solid phase containing the solid phase material complexed with any excess labeled substance and a liquid phase containing the material sought to be quantified which is complexed to the labeled substance.

The liquid phase is then measured for amount of the material sought to be quantified, i.e., analyte or protein. Generally this is accomplished by extracting the liquid phase by syringe, suction, or other means. An appropriate immunoassay format is then employed to measure the amount of labeled substance consistent with the particular label employed.

Typical labeling means can include enzymes, radioisotopes, chromophores, fluorophores or any substance which is capable of generating a detectable signal, either alone or in combination with other reagents. Procedures and methods for labeling and identifying the labeled complexes are well known in the art of diagnostic immunoassay as is generally discussed in L. Miles and C. Hales, Labeled Antibodies and Immunological Assay Systems, Nature 219, 187-189 (1968) and U.S. Patent 3,654,090.

The subject method is especially useful in an automated diagnostic immunoassay apparatus because of its relatively automatic purification of the complex to be measured. Particularly, the immunoassay method can be conducted by mixing the biological fluid to be analyzed with sufficient labeled substance and then adding this reaction mixture to a vessel containing the subject solid phase material or adding the subject solid phase material to the initial reaction mixture wherein the subject solid phase material purifies the reaction mixture of excess labeled substance without requiring additional physical or chemical treatment steps. Thus, the subject method avoids the necessity to centrifuge, prepare an elute from a column, or other more tedious steps to obtain the labeled substance for final measurement.

Example I

A method for measurement of both small or large analytes in a sample fluid. The assay format consisting of preincubation of excess enzyme-labeled antibody with sample such that sample is quantitatively and rapidly bound to form an analyte-enzyme-labeled antibody conjugate. An aliquot of this mixture is transferred to a vessel containing a solid phase material consisting of an analyte covalently coupled to a polymeric bead having a characteristic density to be easily suspended in solution yet sufficiently dense to rapidly sediment by gravity. The solid phase material rapidly captures the excess unconjugated enzyme-labeled antibody and sediments. Following the capture reaction and sedimentation an aliquot containing analyte-enzyme-labeled antibody is transferred to a cuvette for quantitation of enzyme using a fluorogenic substrate. The signal generated is proportional to the analyte concentration in the sample.

Example II

a) Preparation of Enzyme Labeled Antibody

An enzyme labeled antibody of $\beta$-galactosidase conjugated to rabbit anti-digoxin F(ab')$_2$ was prepared. Rabbit antiserum against digoxin was fractionated by means of (NH$_4$)$_2$SO$_4$ and affinity chromatography on agarose-bovine serum albumin-oubain. The resulting digoxin specific antibody was reacted with pepsin to produce F(ab')$_2$ (antibody fragments). This was coupled to E-coli-$\beta$-galactosidase according to the procedure disclosed by Kitagawa and Aikawa, J. Biochem. 79:233 (1976). F(ab)$_2$-$\beta$-galactisidase was isolated by column chromatography.

b) Preparation of Solid Phase Material

Bovine serum albumin-digoxin was prepared as disclosed in PNAS 57:71 (1967). The product was dialyzed against a 0.1M Hepes buffer (7.5 pH) and concentrated to give a 5 mg/ml solution. An 8 ml aliquot of this solution was added to 15 ml of 300 $\mu$m agarose bead having an average diameter of 300 $\mu$m (purchased as Bio-Rad Affi-gel 15 from Bio-Rad Laboratories). The resulting agarose-bovine serum albumin-digoxin beads were washed with 4 molar guanidine-hydrochloric acid and phosphate buffered saline.

Serum solutions containing 0, 1.0, 2.0, 3.0 and 5.0 nanograms per milliliter of digoxin were each tested following the same assay protocol as described below. A control was also run with zero digoxin and no solid phase material. To a 0.05 ml of serum sample was mixed 1 microliter of the enzyme labeled antibody as prepared (a), above. The reaction mixture was incubated at 34°C for ten minutes and then a 20 microliter aliquot was added to 100 microliter solution of the solid phase material (20:1 ratio) prepared from (b), above, with a 0.01 M phosphate buffer, pH 7.4. The mixture was incubated at 34° for 30 minutes with mixing.

The mixture was allowed to briefly rest whereupon it separated into a liquid and solid phase. The $\beta$-galactosidase activity of the supernatant was measured by quantitating the production of fluorescein. The measurement was conducted with a 10 micromolar solution of di-($\beta$-D-galactosyl) fluorescein as a substrate and an assay buffer containing 0.1 M sodium phosphate, bovine gamma globulin, and 0.1% sodium azide at a pH of 7.4.

The measurements were as follows:

| Test No. | Digoxin Concentration (nanograms/ml) | Measured Fluorescence Rate |
|---|---|---|
| Control (no solid phase material) | 0.0 | 1370 |
| 1 | 0.0 | 95 |
| 2 | 1.0 | 110 |
| 3 | 2.0 | 125 |
| 4 | 3.0 | 170 |
| 5 | 5.0 | 245 |

The measurements obtained show that by employing the subject method a standard curve can be made to analyze serum solutions containing unknown digoxin concentrations. Also, the zero concentration measurement as compared to the control shows that the excess antibody conjugate was effectively removed by the subject method.

### Example 3

a) Preparation of F[ab']$_2$-Galactosidase Conjugates

Specific human chorionic gonadotropin (hCG) antibodies were immunopurified from rabbit antibodies (IgG), that were prepared by successive ammonium sulfate precipitations of anti-hCG rabbit sera, by using an hCG-immunoadsorbant. The hCG-immunoadsorbant was prepared by immobilizing hcg on Affi-Gel® 15 using conventional procedures that are described in the Bio-Rad literature available with Aff-Gel® 15.

For immunopurification the IgG was loaded on the hcg-Affi-Gel® 15 column that had been previously washed with 4.0 M Guanidine HCl, 1.0 M acetic acid and tris-buffered saline (TBS). The column was then washed extensively with TBS and eluted with 1.0 M acetic acid. The purified hcG specific antibodies obtained in the elute were extensively dialyzed against 0.1 M sodium acetate pH 4.5.

Fragments [F(ab')$_2$] of the hCG specific antibodies were prepared by digesting the said antibodies with pepsin and then isolating the F(ab')$_2$ fragments from the digestion mixture by column chromatography.

In order to prepare F(ab')$_2$-galactosidase conjugates the F(ab')$_2$ fragments were reacted with m-maleimidobenzoic acid N-hydroxysuccinimide ester (MBS). The resulting MBS derivitized fragments were purified by column chromatography and then subsequently reacted with E. coli $\beta$-galactosidase that had been previously purified by column chromatography. The F(ab')$_2$-galactosidase conjugates produced were isolated from unreacted galactosidase and unreacted F(ab')$_2$ by size exclusice chromatography.

b) Synthesis of hcg solid phase reagent

The hCG-solid phase reagent was prepared by coupling hcg to Tisacryl® GF-2000LS.

Trisacryl is a porous, spherical, polyacrylamide solid phase matrix available from the LKB Corporation, Gaithersburg, Maryland. Prior to coupling with hCG the Trisacryl was activating by extensive washing with water, ethanol, acetone and dimethylformamide (DMF) and then reacting the Trisacryl with carbonyl diimidizole (CDI) in DMF. The activated Trisacryl was then coupled to hcg by the reaction of hcg with the said material in aqueous buffer (pH 10.2). Following the coupling unreacted hcg was removed from the Trisacryl by extensive washing with 1.0 M acetic acid and neutral pH buffers.

c) Analysis of sereum hCG levels

Serum solutions containing known amounts of hCG were used to generate a standard curve according to the following protocol: 25 $\mu$l of F(ab')$_2$-galactosidase, synthesized in (A) above, was added to 100 $\mu$l of sample and 50 $\mu$l TDx buffer. The reaction mixture was incubated at 34 degrees C for 2 minutes 20 seconds and then 50 $\mu$l of the reaction mixture, along with 60 $\mu$l of TDx buffer, was added to a slurry that consisted of 50 $\mu$l of hCG-trisacryl, synthesized in (B) above, and 10 $\mu$l of TDx buffer. This mixture was incubated at 34° C for 10 minutes with mixing. The mixture was allowed to briefly rest whereupon it separated into a liquid and solid phase. The galactosidase activity of the supernatant was measured by quantitating the production of fluorescein. The measurement was conducted with 10 micromolar solution of di($\beta$-D-galactosyl) fluorescein as a substrate and an assay buffer containing 0.1 M sodium phosphate bovine gamma globulin, and 0.1% sodium azide at a pH of 7.4.

Table 1 shows the sensitivity obtained with various lots of hCG-Trisacryl. The results indicate that the sensitivity obtained is not critically dependent on the amount of hcg coupled to the Trisacryl in the 454-50 IU/ml range.

Figure 1 shows the results for fifty clinical samples (N = 50) that were measured for hcg content using a reference method (Abbott $\beta$-HCG 15/15) and the subject method. A correlation coefficient of R = 0.92 and a slope of 0.86 were obtained from analysis of the date shown in Figure 1. These results indicate that the subject method is accurately measuring serum levels of hCG.

Table 1
Comparison of Sensitivity of Various Lots
of hCG-trisacryls

| Lot No. | hCG Coupled to Trisacryl (IU/ml) | Sensitivity (mIU/ml) |
|---------|----------------------------------|----------------------|
| A | 454 | 10.0 |
| B | 151 | 10.0 |
| C | 50 | 12.5 |
| D | 17 | 25.0 |

**Claims**

1. A method for conducting a diagnostic immunoassay comprising the steps of:
    a) forming, in liquid phase, a reaction mixture of a test sample with a molar excess of labeled antibody whereby said labeled antibody is capable of forming a complex with analyte present in said test sample;
    b) contacting said reaction mixture with a solid phase material having immobilized thereon a compound in an amount capable of complexing with any of said excess labeled antibody, to form a solid phase complex;
    c) separating said liquid phase from said solid phase material and any complexes thereof; and
    d) measuring the amount of complex present in said liquid phase,
      characterized in that said solid phase material has a rate of settling by gravity in the range of 5 seconds to 2 minutes per centimeter in water and said separation of step (c) is accomplished by settling of the solid phase material by gravity.

2. The method of Claim 1 wherein said solid phase material has a diameter of from 5 to 300 $\mu$m.

3. The method of Claim 1 wherein said solid phase material comprises a polymeric bead or matrix.

4. The method of Claim 1 wherein said solid phase material is formed from agarose, polystyrene, polyacrylamide, their derivatives or mixtures thereof.

5. The method of Claim 1 wherein said labeled antibody is an enzyme labeled antibody.

6. The method of Claim 1 wherein said compound immobilized on said solid phase and capable of complexing any excess labeled antibody is a corresponding antigen or chemical analogue thereof.

**Patentansprüche**

1. Verfahren zur Durchführung eines diagnostischen Immunoassays, welches die folgenden Stufen umfaßt:
    a) Ausbilden, in flüssiger Phase, eines Reaktionsgemisches einer Testprobe mit einem molaren Überschuß von markiertem Antikörper, wobei dieser markierte Antikörper befähigt ist, mit dem in dieser Testprobe vorhandenen Analyten einen Komplex zu bilden;
    b) Inberührungbringen des Reaktionsgemisches mit einem Festphasenmaterial, auf welchem eine Verbindung in einer Menge immobilisiert ist, welche Menge befähigt ist, jeglichen überschüssigen markierten Antikörper unter Bildung eines Festphasenkomplexes komplex zu binden;
    c) Abtrennen der flüssigen Phase von dem Festphasenmaterial und jeglichen darauf befindlichen Komplexen; und
    d) Messen der in der flüssigen Phase vorliegenden Menge von Komplexen, dadurch gekennzeichnet, daß das Festphasenmaterial eine Absetzgeschwindigkeit unter der Einwirkung der Schwerkraft in einem Bereich von 5 s bis 2 min je cm in Wasser aufweist, und daß das Abtrennen der Stufe (c) durch Absetzenlassen des Festphasenmaterials unter der Einwirkung der Schwerkraft durchgeführt wird.

EP 0 253 270 B1

**2.** Verfahren nach Anspruch 1, wobei das Festphasenmaterial einen Durchmesser von 5 bis 300 $\mu$m aufweist.

**3.** Verfahren nach Anspruch 1, wobei das Festphasenmaterial aus Polymerperlen oder aus einer Polymermatrix besteht.

**4.** Verfahren nach Anspruch 1, wobei das Festphasenmaterial aus Agarose, Polystyrol, Polyacrylamid oder Derivaten oder Gemischen hievon gebildet ist.

**5.** Verfahren nach Anspruch 1, wobei der markierte Antikörper ein mit einem Enzym markierter Antikörper ist.

**6.** Verfahren nach Anspruch 1, wobei die auf der festen Phase immobilisierte Verbindung, welche befähigt ist, jeglichen Überschuß der markierten Antikörpers komplex zu binden, ein entsprechendes Antigen oder ein chemisches Analogon hievon ist.

**Revendications**

**1.** Procédé pour réaliser un essai immunologique de diagnostic comprenant les étapes de :
a) formation, en phase liquide, d'un mélange réactionnel d'un échantillon à étudier avec un excès molaire d'anticorps marqué, ledit anticorps marqué étant capable de former un complexe avec une substance à analyser présente dans ledit échantillon d'essai ;
b) contact dudit mélange réactionnel avec une matière en phase solide sur laquelle est immobilisé un composé en une quantité capable de se complexer avec tout excès d'anticorps marqué pour former un complexe en phase solide ;
c) séparation de ladite phase liquide d'avec ladite matière en phase solide et de tous leurs complexes ; et
d) mesure de la quantité de complexe présente dans ladite phase liquide,
caractérisé en ce que ladite matière en phase solide a une vitesse de sédimentation sous l'effet de la pesanteur dans la gamme de 5 secondes à 20 minutes/cm dans l'eau et ladite séparation de l'étape c) est effectuée par sédimentation de la matière en phase solide sous l'effet de la pesanteur.

**2.** Procédé selon la revendication 1, dans lequel ladite matière en phase solide a un diamètre de 5 à 300 $\mu$m.

**3.** Procédé selon la revendication 1, dans lequel ladite matière en phase solide est constituée d'une perle ou matrice de polymère.

**4.** Procédé selon la revendication 1, dans lequel ladite matière en phase solide est formée à partir d'agarose, de polystyrène, de polyacrylamide, de leurs dérivés ou de leurs mélanges.

**5.** Procédé selon la revendication 1, dans lequel ledit anticorps marqué est un anticorps marqué avec une enzyme.

**6.** Procédé selon la revendication 1, dans lequel ledit composé immobilisé sur ladite phase solide et capable de se complexer avec tout excès d'anticorps marqué est un antigène correspondant ou un analogue chimique de celui-ci.

7

Figure 1.    Correlation of Subject Method with
ABBOTT ß-hCG 15/15

[hCG]  mIU/ml
ABBOTT  ß-hCG 15/15